(19)

Europäisches
Patentamt

European
Patent Office

Office européen
des brevets

(11)     **EP 4 335 368 B1**

(12)                          **EUROPEAN PATENT SPECIFICATION**

(45) Date of publication and mention
of the grant of the patent:
**15.04.2026  Bulletin 2026/16**

(51) International Patent Classification (IPC):
*A61B 5/145* (2006.01)     *A61B 5/01* (2006.01)
*G16H 10/40* (2018.01)     *G16H 40/40* (2018.01)
*G16H 40/63* (2018.01)     *A61B 5/1495* (2006.01)
*A61B 5/1455* (2006.01)     *A61B 5/1459* (2006.01)

(21) Application number: **23196483.4**

(22) Date of filing: **11.09.2023**

(52) Cooperative Patent Classification (CPC):
**A61B 5/14532; A61B 5/01; A61B 5/1451;
A61B 5/1455; A61B 5/1495; G16H 10/40;
G16H 40/40; G16H 40/63; G16H 50/20;**
A61B 5/1459; A61B 2560/0223; G16H 20/17;
G16H 40/67

(54) **SYSTEMS FOR REDUCING DIFFERENCE BETWEEN CALCULATED AND MEASURED ANALYTE
LEVELS**

SYSTEME ZUR VERRINGERUNG DER DIFFERENZ ZWISCHEN BERECHNETEN UND
GEMESSENEN ANALYTKONZENTRATIONEN

SYSTÈMES DE RÉDUCTION DE DIFFÉRENCE ENTRE DES NIVEAUX D'ANALYTES MESURÉS ET
CALCULÉES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **12.09.2022  US 202217931359**

(43) Date of publication of application:
**13.03.2024  Bulletin 2024/11**

(73) Proprietor: **Senseonics, Incorporated
Germantown, MD 20876 (US)**

(72) Inventors:
• **Turksoy, Kamuran
Germantown 20876 (US)**
• **Gosh-Dastidar, Samanwoy
Germantown 20876 (US)**
• **Jacquin, Arnaud
Germantown 20876 (US)**

(74) Representative: **Weickmann & Weickmann
PartmbB
Postfach 860 820
81635 München (DE)**

(56) References cited:
EP-A1- 2 710 951          WO-A1-2021/108610
US-A1- 2005 187 720

• **PAOLO ROSSETTI ET AL: "Estimating Plasma
Glucose from Interstitial Glucose: The Issue of
Calibration Algorithms in Commercial
Continuous Glucose Monitoring Devices",**
SENSORS, vol. 10, no. 12, 3 December 2010
(2010-12-03), pages 10936 - 10952, XP055135973,
DOI: 10.3390/s101210936
• **ANONYMOUS: "Metabolic States of the Body |
Anatomy and Physiology II", 22 August 2022
(2022-08-22), XP093094747, Retrieved from the
Internet <URL:https://web.archive.org/web/
20220822132956/https://courses.lumenlearning.
com/suny-ap2/chapter/
metabolic-states-of-the-body/> [retrieved on
20231024]**

**EP 4 335 368 B1**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

- ANONYMOUS: "6. Glycemic Targets: Standards of Medical Care in Diabetes-2022 | Diabetes Care | American Diabetes Association", DIABETES CARE, vol. 45, no. Supplement_1, 1 January 2022 (2022-01-01), US, pages S83 - S96, XP093218423, ISSN: 0149-5992, Retrieved from the Internet <URL:https://diabetesjournals.org/care/article/45/Supplement_1/S83/138927/6-Glycemic-Targets-Standards-of-Medical-Care-in> [retrieved on 20241101], DOI: 10.2337/dc22-S006

- Y. LEAL ET AL: "Real-Time Glucose Estimation Algorithm for Continuous Glucose Monitoring Using Autoregressive Models", JOURNAL OF DIABETES SCIENCE AND TECHNOLOGY, vol. 4, no. 2, 1 March 2010 (2010-03-01), pages 391 - 403, XP055135975, ISSN: 1932-2968, DOI: 10.1177/193229681000400221

**Description**

**BACKGROUND**

Field of Invention

The present invention relates to glucose monitoring systems. More specifically, aspects of the present disclosure relate to reducing the error between calculated glucose levels and impractical glucose measurements using practical glucose measurements as reference measurements for calibration. Even more specifically, aspects of the present disclosure relate to reducing the error between calculated blood glucose levels and venous blood glucose measurements using capillary blood glucose measurements as reference measurements for calibration.

Discussion of the Background

The prevalence of diabetes mellitus continues to increase in industrialized countries, and projections suggest that this figure will rise to 4.4% of the global population (366 million individuals) by the year 2030. Glycemic control is a key determinant of long-term outcomes in patients with diabetes, and poor glycemic control is associated with retinopathy, nephropathy and an increased risk of myocardial infarction, cerebrovascular accident, and peripheral vascular disease requiring limb amputation. Despite the development of new insulins and other classes of antidiabetic therapy, roughly half of all patients with diabetes do not achieve recommended target hemoglobin A1c (HbA1c) levels < 7.0%.

Frequent self-monitoring of blood glucose (SMBG) is necessary to achieve tight glycemic control in patients with diabetes mellitus, particularly for those requiring insulin therapy. However, current blood (finger-stick) glucose tests are burdensome, and, even in structured clinical studies, patient adherence to the recommended frequency of SMBG decreases substantially over time. Moreover, finger-stick measurements only provide information about a single point in time and do not yield information regarding intraday fluctuations in blood glucose levels that may more closely correlate with some clinical outcomes.

Continuous glucose monitors (CGMs) have been developed in an effort to overcome the limitations of finger-stick SMBG and thereby help improve patient outcomes. These systems enable increased frequency of glucose measurements and a better characterization of dynamic glucose fluctuations, including episodes of unrealized hypoglycemia. Furthermore, integration of CGMs with automated insulin pumps allows for establishment of a closed-loop "artificial pancreas" system to more closely approximate physiologic insulin delivery and to improve adherence.

Monitoring real-time analyte measurements from a living body via wireless analyte monitoring sensor(s) may provide numerous health and research benefits. There is a need to enhance such analyte monitoring systems via innovations. In particular, improved calibration systems and methods are needed for more accurate analyte monitoring.

WO2021/108610 A1 (SENSEONICS INC [US]) 3 June 2021 (2021-06-03) discloses systems that relate to reducing the error between calculated analyte levels and impractical analyte measurements using practical analyte measurements as reference measurements for calibration and more specifically, systems that relate to reducing the error between calculated blood analyte levels and venous blood analyte measurements using capillary blood analyte measurements as reference measurements for calibration. PAOLO ROSSETTI ET AL: "Estimating Plasma Glucose from Interstitial Glucose: The Issue of Calibration Algorithms in Commercial Continuous Glucose Monitoring Devices", SENSORS, vol. 10, no. 12, 3 December 2010 (2010-12-03), pages 10936-10952, XP055135973, discloses systems for estimating plasma glucose from interstitial glucose and calibration algorithms in commercial continuous glucose monitoring devices. EP2710951 A1 (UNIV VALENCIA POLITECNICA [ES]) 26 March 2014 (2014-03-26) discloses a plasma glucose estimation method and continuous monitoring of analytes measured in compartments alternative to those that are traditionally used. US2005/187720 A1 (GOODE PAUL V JR [US] ET AL) 25 August 2005 (2005-08-25) discloses systems for updating the calibration in analyte sensor data processing.

**SUMMARY**

The invention is defined by the appended claims

BRIEF DESCRIPTION OF THE DRAWINGS

The accompanying drawings, which are incorporated herein and form part of the specification, illustrate various, non-limiting aspects of the present disclosure. In the drawings, like reference numbers indicate identical or functionally similar elements.

FIG. 1 is a schematic view illustrating an analyte monitoring system embodying aspects of the present disclosure.

FIG. 2 is a schematic view illustrating a sensor and transceiver of an analyte monitoring system embodying aspects of the present disclosure.

FIG. 3 is a schematic view illustrating a transceiver embodying aspects of the present disclosure.

FIG. 4 is an error diagram showing errors between calculated blood glucose levels, capillary blood glucose level measurements, venous blood glucose level measurements, and ground truth blood glucose levels.

FIG. 5 illustrates an exemplary error model between capillary blood glucose level measurements and venous blood glucose level measurements.

FIG. 6 is a flow chart illustrating a calibration process embodying aspects of the present disclosure.

FIGS. 7A and 7B are flow charts illustrating a calibration process embodying aspects of the present disclosure.

FIG. 8 is a flow chart illustrating a calibration process embodying aspects of the present disclosure.

FIG. 9 illustrates a computer of a transceiver embodying aspects of the present disclosure.

DETAILED DESCRIPTION OF PREFERRED EMBODIMENTS

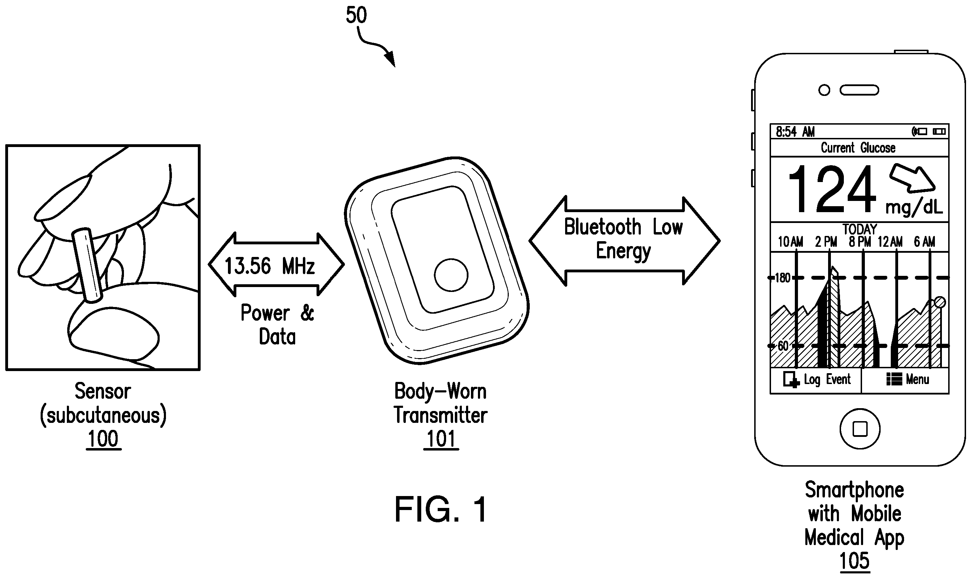

FIG. 1 is a schematic view of an exemplary analyte monitoring system 50 embodying aspects of the present disclosure. The analyte monitoring system 50 may be a continuous analyte monitoring system (*e.g.,* a continuous glucose monitoring system). In some aspects, the analyte monitoring system 50 may include one or more of an analyte sensor 100, a transceiver 101, and a display device 105. In some aspects, the sensor 100 may be small, fully subcutaneously implantable sensor that measures analyte (*e.g.,* glucose) levels in a medium (*e.g.,* interstitial fluid) of a living animal (*e.g.,* a living human). However, this is not required, and, in some alternative aspects, the sensor 100 may be a partially implantable (*e.g.,* transcutaneous) sensor or a fully external sensor. In some aspects, the transceiver 101 may be an externally worn transceiver (*e.g.,* attached via an armband, wristband, waistband, or adhesive patch). In some aspects, the transceiver 101 may remotely power and/or communicate with the sensor to initiate and receive the measurements (*e.g.,* via near field communication (NFC)). However, this is not required, and, in some alternative aspects, the transceiver 101 may power and/or communicate with the sensor 100 via one or more wired connections. In some aspects, the transceiver 101 may be a smartphone (*e.g.,* an NFC-enabled smartphone). In some aspects, the transceiver 101 may communicate information (*e.g.,* one or more glucose levels) wirelessly (*e.g.,* via a Bluetooth™ communication standard such as, for example and without limitation Bluetooth Low Energy) to a hand held application running on a display device 105 (*e.g.,* smartphone). In some aspects, the analyte monitoring system 50 may include a web interface for plotting and sharing of uploaded data.

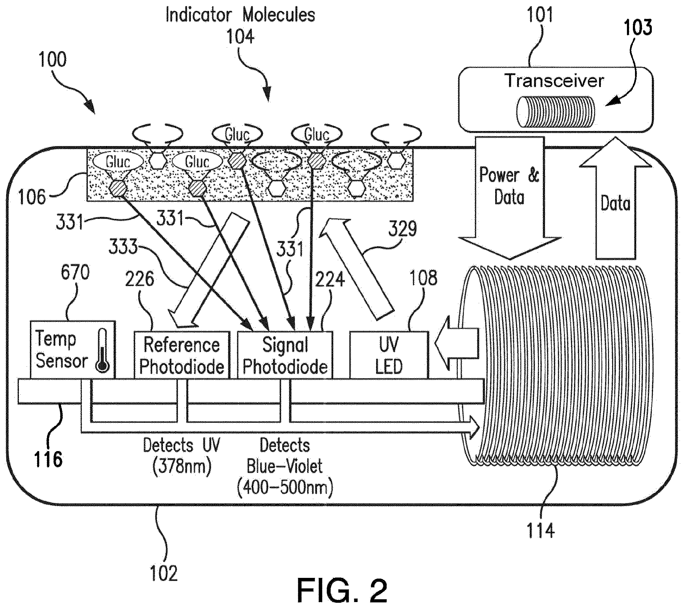

In some aspects, as illustrated in FIG. 2, the transceiver 101 may include an inductor 103, such as, for example, a coil. The transceiver 101 may generate an electromagnetic wave or electrodynamic field (*e.g.,* by using a coil) to induce a current in an inductor 114 of the sensor 100, which powers the sensor 100. The transceiver 101 may also convey data (*e.g.,* commands) to the sensor 100. For example, in some aspects, the transceiver 101 may convey data by modulating the electromagnetic wave used to power the sensor 100 (*e.g.,* by modulating the current flowing through a coil 103 of the transceiver 101). The modulation in the electromagnetic wave generated by the transceiver 101 may be detected/extracted by the sensor 100. Moreover, the transceiver 101 receives sensor data (*e.g.,* measurement information) from the sensor 100. For example, in some aspects, the transceiver 101 may receive sensor data by detecting modulations in the electromagnetic wave generated by the sensor 100, (*e.g.,* by detecting modulations in the current flowing through the coil 103 of the transceiver 101).

The inductor 103 of the transceiver 101 and the inductor 114 of the sensor 100 may be in any configuration that permits adequate field strength to be achieved when the two inductors are brought within adequate physical proximity.

In some aspects, as illustrated in FIG. 2, the sensor 100 may be encased in a sensor housing 102 (*e.g.,* body, shell, capsule, or encasement), which may be rigid and biocompatible. The sensor 100 may include an analyte indicator 106, such as, for example, a polymer graft coated, diffused, adhered, or embedded on or in at least a portion of the exterior surface of the sensor housing 102. The analyte indicator 106 (*e.g.,* polymer graft) of the sensor 100 may include indicator molecules 104 (*e.g.,* fluorescent indicator molecules) exhibiting one or more detectable properties (*e.g.,* optical properties) based on the amount or concentration of the analyte in proximity to the analyte indicator 106. In some aspects, the sensor 100 may include a light source 108 that emits excitation light 329 over a range of wavelengths that interact with the indicator molecules 104. The sensor 100 may also include one or more photodetectors 224, 226 (*e.g.,* photodiodes, phototransistors, photoresistors, or other photosensitive elements). The one or more photodetectors (*e.g.,* photodetector 224) may be sensitive to emission light 331 (*e.g.,* fluorescent light) emitted by the indicator molecules 104 such that a signal generated by a photodetector (*e.g.,* photodetector 224) in response thereto is indicative of the level of emission light 331 of the indicator molecules and, thus, the amount or concentration of the analyte of interest (*e.g.,* glucose). In some non-limiting aspects, one or more of the photodetectors (*e.g.,* photodetector 226) may be sensitive to excitation light 329 that is reflected from the analyte indicator 106 as reflection light 333. In some aspects, one or more of the photodetectors may be covered by one or more filters that allow only a certain subset of wavelengths of light to pass through (*e.g.,* a subset of wavelengths corresponding to emission light 331 or a subset of wavelengths corresponding to reflection light 333) and reflect the remaining wavelengths. In some aspects, the sensor 100 may include a temperature transducer 670. In some aspects, the sensor 100 may include a drug-eluting polymer matrix that disperses one or more therapeutic agents (*e.g.,* an anti-inflammatory drug).

In some aspects, as illustrated in FIG. 2, the sensor 100 may include a substrate 116. In some aspects, the substrate 116 may be a circuit board (*e.g.,* a printed circuit board (PCB) or flexible PCB) on which circuit components (*e.g.,* analog and/or digital circuit components) may be mounted or otherwise attached. However, in some alternative aspects, the substrate 116 may be a semiconductor substrate having circuitry fabricated therein. The circuitry may include analog and/or digital circuitry. Also, in some semiconductor substrate aspects, in addition to the circuitry fabricated in the semiconductor substrate, circuitry may be mounted or otherwise attached to the semiconductor substrate 116. In other words, in some semiconductor substrate aspects, a portion or all of the circuitry, which may include discrete circuit elements, an integrated circuit (*e.g.,* an application specific integrated circuit (ASIC)) and/or other electronic components (*e.g.,* a non-volatile memory), may be fabricated in the semiconductor substrate 116 with the remainder of the circuitry is secured to the semiconductor substrate 116 and/or a core (*e.g.,* ferrite core) for the inductor 114. In some aspects, the semiconductor substrate 116 and/or a core may provide communication paths between the various secured components.

In some aspects, the one or more of the sensor housing 102, analyte indicator 106, indicator molecules 104, light source 108, photodetectors 224, 226, temperature transducer 670, substrate 116, and inductor 114 of sensor 100 may include some or all of the features described in one or more of U.S. Application Serial No. 13/761,839, filed on February 7, 2013, U.S. Application Serial No. 13/937,871, filed on July 9, 2013, and U.S. Application Serial No. 13/650,016, filed on October 11, 2012. Similarly, the structure and/or function of the sensor 100 and/or transceiver 101 may be as described in one or more of U.S. Application Serial Nos. 13/761,839, 13/937,871, and 13/650,016.

Although in some aspects, as illustrated in FIG. 2, the sensor 100 may be an optical sensor, this is not required, and, in one or more alternative aspects, sensor 100 may be a different type of analyte sensor, such as, for example, an electrochemical sensor, a diffusion sensor, or a pressure sensor. Also, although in some aspects, as illustrated in FIGS. 1 and 2, the analyte sensor 100 may be a fully implantable sensor, this is not required, and, in some alternative aspects, the sensor 100 may be a transcutaneous sensor having a wired connection to the transceiver 101. For example, in some alternative aspects, the sensor 100 may be located in or on a transcutaneous needle (*e.g.,* at the tip thereof). In these aspects, instead of wirelessly communicating using inductors 103 and 114, the sensor 100 and transceiver 101 may communicate using one or more wires connected between the transceiver 101 and the transceiver transcutaneous needle that includes the sensor 100. For another example, in some alternative aspects, the sensor 100 may be located in a catheter (*e.g.,* for intravenous blood glucose monitoring) and may communicate (wirelessly or using wires) with the transceiver 101.

In some aspects, the sensor 100 may include a transceiver interface. In some aspects where the sensor 100 includes an antenna (*e.g.,* inductor 114), the transceiver interface may include the antenna (*e.g.,* inductor 114) of sensor 100. In some of the transcutaneous aspects where there exists a wired connection between the sensor 100 and the transceiver 101, the transceiver interface may include the wired connection.

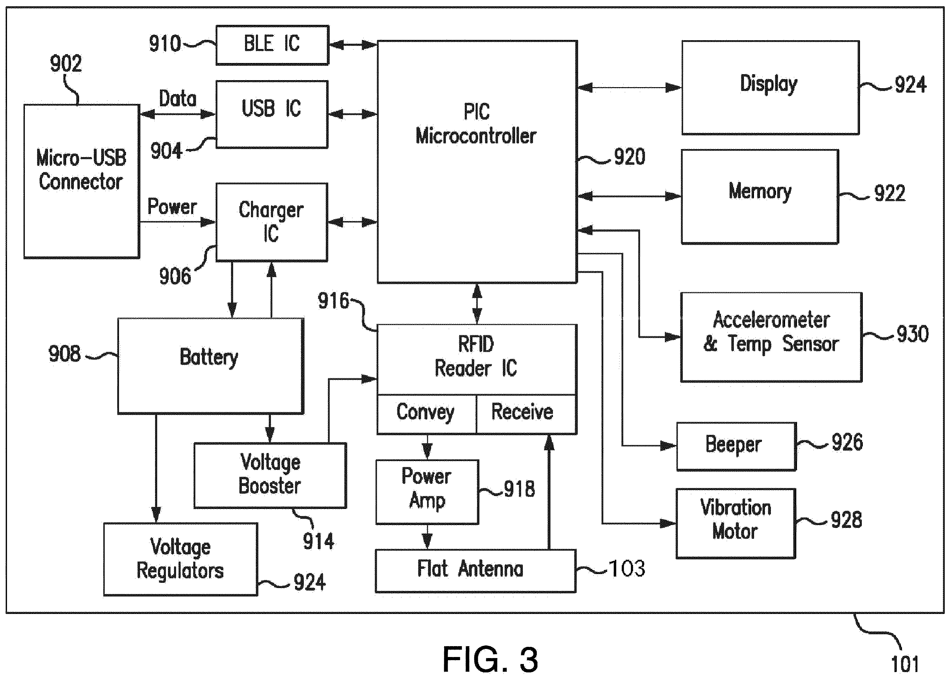

FIG. 3 is a schematic view of an external transceiver 101 according to a non-limiting aspect. In some aspects, the transceiver 101 may have a connector 902, such as, for example, a Micro-Universal Serial Bus (USB) connector. The connector 902 may enable a wired connection to an external device, such as a personal computer (*e.g.,* personal computer 109) or a display device 105 (*e.g.,* a smartphone).

The transceiver 101 may exchange data to and from the external device through the connector 902 and/or may receive power through the connector 902. The transceiver 101 may include a connector integrated circuit (IC) 904, such as, for example, a USB-IC, which may control transmission and receipt of data through the connector 902. The transceiver 101 may also include a charger IC 906, which may receive power via the connector 902 and charge a battery 908 (*e.g.,* lithium-polymer battery). In some aspects, the battery 908 may be rechargeable, may have a short recharge duration, and/or may have a small size.

In some aspects, the transceiver 101 may include one or more connectors in addition to (or as an alternative to) Micro-USB connector 904. For example, in some alternative aspects, the transceiver 101 may include a spring-based connector (*e.g.,* Pogo pin connector) in addition to (or as an alternative to) Micro-USB connector 904, and the transceiver 101 may use a connection established via the spring-based connector for wired communication to a personal computer (*e.g.,* personal computer 109) or a display device 105 (*e.g.,* a smartphone) and/or to receive power, which may be used, for example, to charge the battery 908.

In some aspects, the transceiver 101 may have a wireless communication IC 910, which enables wireless communication with an external device, such as, for example, one or more personal computers (*e.g.,* personal computer 109) or one or more display devices 105 (*e.g.,* a smartphone). In some aspects, the wireless communication IC 910 may employ one or more wireless communication standards to wirelessly transmit data. The wireless communication standard employed may be any suitable wireless communication standard, such as an ANT standard, a Bluetooth standard, or a Bluetooth Low Energy (BLE) standard (*e.g.,* BLE 4.0). In some aspects, the wireless communication IC 910 may be configured to wirelessly transmit data at a frequency greater than 1 gigahertz (*e.g.,* 2.4 or 5 GHz). In some aspects, the wireless communication IC 910 may include an antenna (*e.g.,* a Bluetooth antenna). In some aspects, the antenna of the wireless communication IC 910 may be entirely contained within the housing (e.g., housing 206 and 220) of the transceiver 101. However, this is not required, and, in alternative aspects, all or a portion of the antenna of the wireless communication IC 910 may be external to the transceiver housing.

In some aspects, the transceiver 101 may include a display interface, which may enable communication by the transceiver 101 with one or more display devices 105. In some aspects, the display interface may include the antenna of the wireless communication IC 910 and/or the connector 902. In some aspects, the display interface may additionally include the wireless communication IC 910 and/or the connector IC 904.

In some aspects, the transceiver 101 may include voltage regulators 912 and/or a voltage booster 914. The battery 908 may supply power (via voltage booster 914) to radiofrequency identification (RFID) reader IC 916, which uses the inductor 103 to convey information (*e.g.,* commands) to the sensor 101 and receive information (*e.g.,* measurement information) from the sensor 100. In some aspects, the sensor 100 and transceiver 101 may communicate using near field communication (NFC) (*e.g.,* at a frequency of 13.56 MHz). In the illustrated aspect, the inductor 103 is a flat antenna. In some aspects, the antenna may be flexible. However, as noted above, the inductor 103 of the transceiver 101 may be in any configuration that permits adequate field strength to be achieved when brought within adequate physical proximity to the inductor 114 of the sensor 100. In some aspects, the transceiver 101 may include a power amplifier 918 to amplify the signal to be conveyed by the inductor 103 to the sensor 100.

The transceiver 101 may include a computer 920 and a memory 922. In some aspects, the memory 922 (*e.g.,* Flash memory) may be non-volatile and/or capable of being electronically erased and/or rewritten. In some aspects, the computer 920 may include a processor and a non-transitory memory. In some aspects, the computer 920 may be, for example and without limitation, a peripheral interface controller (PIC) microcontroller. The computer 920 may control the overall operation of the transceiver 101. For example, the computer 920 may control the connector IC 904 or wireless communication IC 910 to transmit data via wired or wireless communication and/or control the RFID reader IC 916 to convey data via the inductor 103. The computer 920 may also control processing of data received via the inductor 103, connector 902, or wireless communication IC 910.

In some aspects, the transceiver 101 may include a sensor interface, which may enable communication by the transceiver 101 with a sensor 100. In some aspects, the sensor interface may include the inductor 103. In some aspects, the sensor interface may additionally include the RFID reader IC 916 and/or the power amplifier 918. However, in some alternative aspects where there exists a wired connection between the sensor 100 and the transceiver 101 (*e.g.,* transcutaneous aspects), the sensor interface may include the wired connection.

In some aspects, the transceiver 101 may include a display 924 (*e.g.,* liquid crystal display and/or one or more light emitting diodes), which the computer 920 may control to display data (*e.g.,* glucose levels values). In some aspects, the transceiver 101 may include a speaker 926 (*e.g.,* a beeper) and/or vibration motor 928, which may be activated, for example, in the event that an alarm condition (*e.g.,* detection of a hypoglycemic or hyperglycemic condition) is met. The transceiver 101 may also include one or more additional sensors 930, which may include an accelerometer and/or temperature sensor, which may be used in the processing performed by the computer 920.

In some aspects, the transceiver 101 may be a body-worn transceiver that is a rechargeable, external device worn over the sensor implantation or insertion site. The transceiver 101 may supply power to the proximate sensor 100, calculate glucose levels from data received from the sensor 100, and/or transmit the calculated glucose levels to a display device 105 (see FIG. 1). Power may be supplied to the sensor 100 through an inductive link (*e.g.,* an inductive link of 13.56 MHz). In some aspects, the transceiver 101 may be placed using an adhesive patch or a specially designed strap or belt. The external transceiver 101 may read measured analyte data from a subcutaneous sensor 100 (*e.g.,* up to a depth of 2 cm or more). The transceiver 101 may periodically (*e.g.,* every 2, 5, or 10 minutes) read sensor data and calculate an glucose level and an glucose level trend. From this information, the transceiver 101 may also determine if an alert and/or alarm condition exists, which may be signaled to the user (*e.g.,* through vibration by vibration motor 928 and/or an LED of the transceiver's display 924 and/or a display of a display device 105).

The information from the transceiver 101 (*e.g.,* calculated glucose levels, calculated glucose level trends, alerts, alarms, and/or notifications) may be transmitted to a display device *105* (*e.g.,* via Bluetooth Low Energy with Advanced Encryption Standard (AES)-Counter CBC-MAC (CCM) encryption) for display by a mobile medical application (MMA) being executed by the display device 105. In some aspects, the MMA may provide alarms, alerts, and/or notifications in addition to any alerts, alarms, and/or notifications received from the transceiver 101. In one aspect, the MMA may be configured to provide push notifications. In some aspects, the transceiver 101 may have a power button (*e.g.,* button 208) to allow the user to turn the device on or off, reset the device, or check the remaining battery life. In some aspects, the transceiver 101 may have a button, which may be the same button as a power button or an additional button, to suppress one or more user notification signals (*e.g.,* vibration, visual, and/or audible) of the transceiver 101 generated by the transceiver 101 in response to detection of an alert or alarm condition.

In some aspects, the transceiver 101 of the analyte monitoring system 120 may receive one or more sensor measurements indicative of an amount, level, or concentration of an analyte in a first medium (e.g., interstitial fluid ("ISF")) in proximity to the analyte sensor 100. In some aspects, the transceiver 101 may receive the sensor measurements from the sensor 100 periodically (e.g., every 1, 2, 5, 10, 15, or 20 minutes). In some aspects, the one or more sensor measurements may include, for example and without limitation, one or more of (i) one or more measurements indicative of an amount of emission light from indicator molecules 104 of the analyte indicator 106 of the sensor 100 (e.g., as measured by one or more photodetectors 224 of the sensor 100), (ii) one or more measurements indicative of an amount of reference light (e.g., as measured by one or more photodetectors 226 of the sensor 100), and (iii) one or more temperature measurements (e.g., as measured by one or more temperature transducers 670 of the sensor 100). The transceiver 101 uses the received sensor measurements to calculate a first medium glucose level (e.g., an ISF glucose level).

In some aspects, the transceiver 101 may use the calculated first medium glucose level and at least one or more previously calculated first medium glucose levels to calculate a rate of change of the first medium glucose level ("M1_ROC"). In some aspects, to calculate M1_ROC, the transceiver 101 may use just the calculated first medium glucose level and the most recent previously calculated first medium glucose level and determine M1_ROC as the difference between the calculated first medium glucose level and most recent previously calculated first medium glucose level divided by the time difference between a time stamp for the calculated first medium glucose level and a time stamp for the most recent previously calculated first medium glucose level. In some alternative aspects, to calculate M1_ROC, the transceiver 101 may use the calculated first medium glucose level and a plurality of the most recent previously calculated first medium glucose levels. In some aspects, the plurality of the most recent previously calculated first medium glucose levels may be, for example and without limitation, the previous two calculated first medium glucose levels, the previous 20 calculated first medium glucose levels, or any number of previously calculated first medium glucose levels in between (e.g., the previous 5 calculated first medium glucose levels). In other alternative aspects, to calculate M1_ROC, the transceiver 101 may use the calculated first medium glucose level and the previously calculated first medium glucose levels that were calculated during a time period. In some aspects, the time period may be, for example and without limitation, the last one minute, the last 60 minutes, or any amount of time in between (e.g., the last 25 minutes). In some aspects where the transceiver 101 uses the calculated first medium glucose level and more than one previously calculated first medium glucose levels to calculate M1_ROC, the transceiver 101 may use, for example, linear or non-linear regression to calculate M1_ROC.

The transceiver 101 may convert the calculated first medium glucose level into a second medium glucose level (i.e., a blood glucose level) by performing a lag compensation, which compensates for the time lag between a second medium glucose level and an first medium glucose level (e.g., the time lag between a blood glucose level and an ISF glucose level). In some aspects, the transceiver 101 may calculate the second medium glucose level using at least the calculated first medium glucose level and the calculated M1_ROC. In some aspects, the transceiver 101 may calculate the second medium glucose level as $M1\_ROC/p_2 + (1+p_3/p_2)*M1\_analyte$, where $p_2$ is analyte diffusion rate, $p_3$ is the analyte consumption rate, and M1_analyte is the calculated first medium glucose level.

In some aspects, the transceiver 101 may store one or more of the calculated first medium glucose level, calculated M1_ROC, and calculated second medium glucose level (*e.g.,* in memory 922). In some aspects, the transceiver 101 may convey the calculated first medium glucose level to the display device 105, and the display device 105 may display the calculated first medium glucose level.

In some aspects, the analyte monitoring system 50 may calibrate the conversion of sensor measurements to second medium (e.g., blood) glucose levels. That is, in some aspects, the analyte monitoring system 50 may calibrate the manner in which the transceiver 101 calculates second medium glucose levels using the sensor measurements. In some aspects, the calibration may be performed approximately periodically (*e.g.,* approximately every 12 or 24 hours). The calibration is performed using one or more reference measurements. The one or more references measurements are capillary blood glucose level measurements (*e.g.,* one or more self-monitoring blood glucose (SMBG) measurements). In some aspects, the display device 105 may prompt a user for one or more reference measurements. In some aspects, the one or more reference measurements may be entered into the analyte monitoring system 50 using a user interface of the display device 105. In some aspects, the display device 105 may convey one or more references measurements to the transceiver 101. In some aspects, the transceiver 101 may receive the one or more reference measurements from the display device 105 and use the one or more reference measurements to perform the calibration.

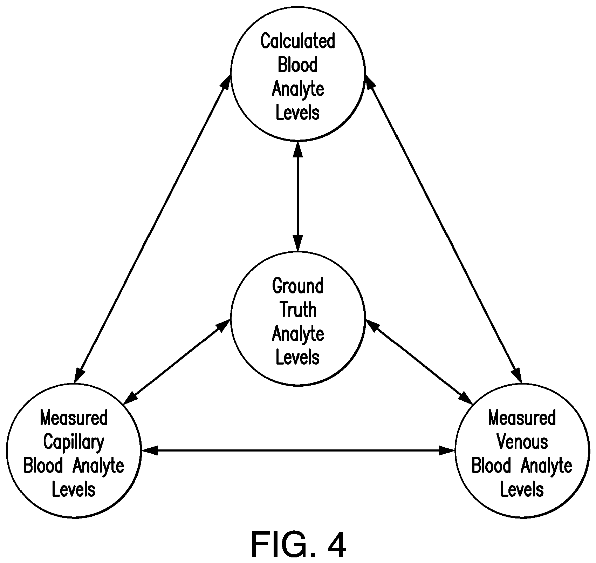

The second medium is venous blood, the reference measurements are capillary blood glucose level measurements, and the transceiver 101 uses sensor measurements received from the sensor 100 to calculate blood glucose levels. FIG. 4 is an error diagram showing errors between each of (i) blood glucose levels calculated by the transceiver 101, (ii) capillary blood glucose level measurements, (iii) venous blood glucose level measurements, and (iv) ground truth blood glucose levels. The ground truth glucose level may represent the actual glucose level of an animal (e.g., human) body. The ground truth glucose levels cannot be measured directly and are never known. Because ground truth glucose levels are not known, venous blood glucose level measurements are often used as the gold standard glucose level measurements for assessing the accuracy of an analyte monitoring system. However, venous blood glucose level measurements are not practical for continuous analyte monitoring because venous blood glucose level measurements are generally only available in a laboratory setting where a technician can find a vein from which venous blood can be withdrawn. In contrast, capillary blood glucose level measurements can be performed by a patient anywhere (e.g., using a finger-stick blood sample, which is relatively painless and simple when compared to a venous blood draw). In some aspects, as shown in FIG. 4, the calculated glucose levels, capillary blood glucose level measurements, and venous blood glucose level measurements may each have their own error profiles with respect to the ground truth glucose levels.

As there are no ground truth glucose level measurements, analyte monitoring systems cannot use ground truth glucose level measurements for calibration purposes. In addition, analyte monitoring systems typically do not use venous blood glucose level measurements for calibration purposes because venous blood glucose level measurements are generally available only in laboratory settings. Instead, analyte monitoring systems typically use capillary blood glucose level measurements, which are generally available in real life settings, for calibration purposes.

In some aspects, the transceiver 101 may calibrate the conversion of sensor measurements to calculated glucose levels using one or more capillary blood glucose level measurements. In some aspects, the calibration using the one or more capillary blood glucose level measurements may minimize the error between calculated blood glucose levels and the capillary blood glucose level measurements. However, due to errors between capillary blood glucose level measurements and venous blood glucose level measurements (see FIG. 4), calibrating the conversation to minimize the error between the calculated blood glucose levels and the capillary blood glucose level measurements may not minimize the error between the calculated blood glucose levels and venous blood glucose level measurements when the accuracy of the calculated blood glucose levels are evaluated in a laboratory setting.

In some alternative aspects, the transceiver 101 may use one or more capillary blood glucose level measurements to calibrate the conversion of sensor measurements to calculated blood glucose levels but calibrate the conversion to minimize the error between calculated blood glucose levels and estimated venous blood glucose levels (as opposed to minimizing the error between calculated blood glucose levels and the capillary blood glucose level measurements). In some aspects, the calibration using one or more capillary blood glucose level measurements may include (i) converting the one or more capillary blood glucose level measurements to one or more estimated venous blood glucose levels and (ii) calibrating the conversion of sensor measurements to calculated blood glucose levels to minimize the error between calculated blood glucose levels and the one or more estimated venous blood glucose levels.

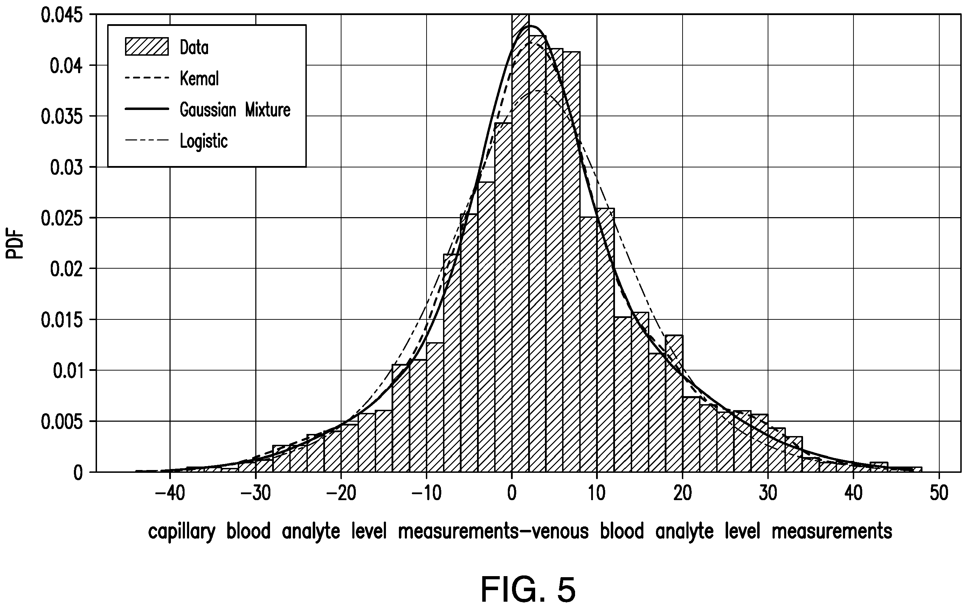

The transceiver 101 converts the one or more capillary blood glucose level measurements to one or more estimated venous blood glucose levels based on a model of the difference between capillary blood glucose level measurements and venous blood glucose level measurements. FIG. 5 illustrates an exemplary model of the difference between capillary blood glucose level measurements and venous blood glucose level measurements based on experimental data collected during one or more analyte monitoring clinics.

In some aspects, the transceiver 101 may convert the one or more capillary blood glucose level measurements to one or more estimated venous blood glucose levels based on a cost function that maximizes the likelihood (or minimizes the negative likelihood) of the fit of the error between calculated blood glucose levels and capillary blood glucose level measurements to the error model between the capillary and venous blood glucose level measurements. Non-limiting examples of a cost function that maximizes the likelihood of the fit of the error and a cost function that minimize the negative likelihood of the fit of the error are shown below. In the non-limiting examples, $\theta$ is the cost function, $CGM_\theta(t)$ is a calculated blood glucose level at a time t, and $SMBG(t)$ is a capillary blood glucose level measurement at a time t.

$$\hat{\theta} = \max_\theta \sum_{t=1}^{N} w_1 \ln(f_1(e_t)) + w_2 \ln(f_2(e_t)) \qquad \hat{\theta} = \min_\theta \sum_{t=1}^{N} -w_1 \ln(f_1(e_t)) - w_2 \ln(f_2(e_t))$$

$$e_t = SMBG(t) - CGM_\theta(t) \qquad\qquad e_t = SMBG(t) - CGM_\theta(t)$$

$$f_j(e_t) = \frac{1}{\sqrt{2\pi\sigma_j{}^2}} e^{-\frac{(e_t-\mu_j)^2}{2\sigma_j{}^2}} \qquad\qquad f_j(e_t) = \frac{1}{\sqrt{2\pi\sigma_j{}^2}} e^{-\frac{(e_t-\mu_j)^2}{2\sigma_j{}^2}}$$

In some aspects, the calibration of sensor measurements to calculated blood glucose levels may minimize the error between the calculated blood glucose levels and estimated venous blood glucose levels using capillary blood glucose level measurements, which unlike venous blood glucose measurements can be obtained practically. In some aspects, the error model may be updated based on one or more new experimental datasets.

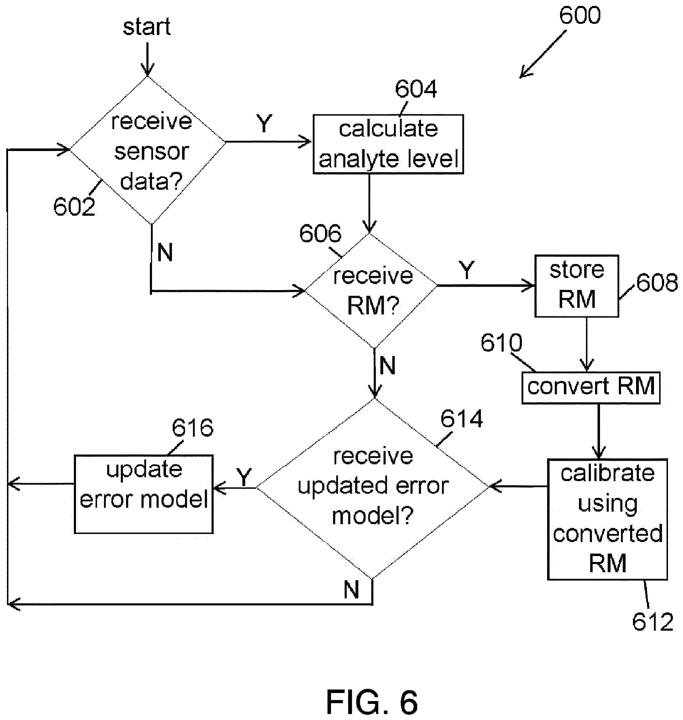

FIG. 6 is a flow chart illustrating a calibration process 600 according to some non-limiting aspects of the invention. In some aspects, the transceiver 101 may perform one or more steps of the calibration process 600. In some non-limiting aspects, the computer 920 of the transceiver 101 may perform one or more steps of the calibration process 600.

In some aspects, as shown in FIG. 6, the calibration process 600 may include a step 602 in which the transceiver 101 determines whether the transceiver 101 has received sensor data (*e.g.,* light and/or temperature measurements) from the sensor 100. In some aspects, the sensor data may be received following a command (*e.g.,* a measurement command or a read sensor data command) conveyed from the transceiver 101 to the sensor 100. However, this is not required, and, in some alternative aspects, the sensor 100 may control when sensor data is conveyed to the transceiver 101, or the sensor 100 may continuously convey sensor data to the transceiver 101. In some aspects, the transceiver 101 may receive the sensor data periodically (*e.g.,* every 1, 2, 5, 10, 15, or 20 minutes). In some aspects, the transceiver 101 may receive the sensor data wirelessly. For example and without limitation, in some aspects, the transceiver 101 may receive the sensor data by detecting modulations in an electromagnetic wave generated by the sensor 100 (*e.g.,* by detecting modulations in the current flowing through the coil 103 of the transceiver 101). However, this is not required, and, in some alternative aspects, the transceiver 101 may receive the sensor data via a wired connection to the sensor 100. In some aspects, if the sensor has received sensor data, the calibration process 600 may proceed from step 602 to an glucose level calculation step 604. In some aspects, if the transceiver 101 has not received sensor data, the calibration process 600 may proceed from step 602 to a step 606.

In some aspects, the calibration process 600 may include the glucose level calculation step 604. In some aspects, the step 604 may include calculating an glucose level using a current conversion function and the received sensor data. In some aspects, the calculated glucose level may be a calculated second medium glucose level (e.g., a calculated blood glucose level). In some aspects, the glucose level calculation step 604 may include calculating a first medium glucose level (e.g., an ISF glucose level), an M1_ROC (e.g., an ISF _ROC), and the second medium glucose level.

In the glucose level calculation step 604, the transceiver 101 calculates the first medium glucose level using the received sensor data. In some aspects, the first medium glucose level may be a measurement of the amount or concentration of the analyte in the first medium (e.g., interstitial fluid) in proximity to the analyte indicator 106. In some aspects, calculation of the first medium glucose level may include, for example and without limitation, some or all of the features described in U.S. Application Serial No. 13/937,871, filed on July 9, 2013.

In some aspects, in the glucose level calculation step 604, the transceiver 101 may calculate the M1_ROC using at least the calculated first medium glucose level. In some aspects, the transceiver 101 may calculate the M1_ROC using at least the calculated first medium glucose level and one or more previously calculated first medium glucose levels (*e.g.,* one or more ISF glucose levels calculated using previously received sensor data).

In some aspects, in the glucose level calculation step 604, the transceiver 101 may calculate the second medium glucose level (e.g., blood glucose level) by performing a lag compensation. In some aspects, the transceiver 101 may calculate the blood glucose level using at least the calculated ISF glucose level and the calculated ISF_ROC. In some aspects, the transceiver 101 may calculate the blood glucose level using the formula $ISF\_ROC/p_2 + (1+p_3/p_2)*ISF\_analyte$, where $p_2$ is the analyte diffusion rate, $p_3$ is the analyte consumption rate, $ISF\_ROC$ is the calculated ISF_ROC, and $ISF\_analyte$ is the calculated ISF glucose level. However, this is not required, and some alternative aspects may use a different formula for calculating the blood glucose level.

In some aspects, in step 604, the transceiver 101 may display the calculated glucose level (e.g., calculated blood glucose level). In some aspects, the transceiver 101 may display the calculated glucose level by conveying it to the display device 105 for display. In some aspects, the transceiver 101 may additionally or alternatively display the calculated glucose level by display it on a display (e.g., display 924) of the transceiver 101.

In some aspects, the calibration process 600 may include the step 606 in which the transceiver 101 determines whether the transceiver 101 has received a reference measurement. The reference measurement is a capillary blood glucose measurement (e.g., an SMBG measurement) obtained from, for example and without limitation, a finger-stick blood sample. In some aspects, the transceiver 101 may receive reference measurements periodically or on an as-needed basis. In some aspects, the transceiver 101 may receive the reference measurement from the display device 105 (e.g., using the display interface of the transceiver 101). In some aspects, the transceiver 101 may cause the display device 105 to prompt a user for the reference measurement, and, in response, the user may enter the reference measurement into the display device 105. In some alternative aspects, the transceiver 101 may prompt a user for the reference measurement, and, in response, the user may enter the reference measurement directly into the transceiver 101.

In some aspects, if the transceiver 101 has not received a reference measurement, the calibration process 600 may proceed to a step 614. In some aspects, if the transceiver 101 has received a reference measurement, the calibration process 600 may proceed to a step 608.

In some aspects, the calibration process 600 may include a step 608 in which the reference measurement is stored, for example, in a calibration point memory (*e.g.,* a circular buffer). In some aspects, the reference measurement may be stored in the calibration point memory with a corresponding reference time stamp. In some aspects, the calibration process 600 may proceed from step 608 to a step 610.

The calibration process 600 includes a step 610 in which the transceiver 101 converts the received reference measurement into an estimated glucose level. The received reference measurement is a capillary blood glucose measurement, and the estimated glucose level is an estimated venous blood glucose level. The conversion of the reference measurement into the estimated glucose level is carried out using a model (e.g., error model) of differences between capillary blood glucose level measurements and venous blood glucose level measurements. In some aspects, the conversion of the reference measurement into the estimated glucose level may be carried out using a cost function that maximizes the likelihood of a fit of the error between calculated glucose levels and capillary blood glucose level measurements to the model of the differences between the capillary blood glucose level measurements and the venous blood glucose level measurements. In some alternative aspects, the conversion of the reference measurement into the estimated glucose level may be carried out using a cost function minimizes the negative likelihood of a fit of the error between calculated glucose levels and capillary blood glucose level measurements to the model of the differences between the capillary blood glucose level measurements and the venous blood glucose level measurements. In some aspects, the transceiver 101 may store the reference measurement (e.g., in a calibration point memory).

In some aspects, the calibration process 600 may include a calibration step 612 in which the transceiver 101 updates the conversion function used to calculate second medium (e.g., blood) glucose levels from the received sensor data. The transceiver 101 updates the conversion function using at least the estimated venous blood glucose level as a calibration point. In some aspects, the transceiver 101 may update the conversion function using the estimated glucose level and one or more additional estimated glucose levels, which were converted from one or more previously received reference measurements, as calibration points. In some aspects, the transceiver 101 may assign weights to the estimated glucose levels (e.g., based on how old the estimated glucose levels are). In some aspects, the updated conversion function minimizes the error between glucose levels calculated using the updated conversion function and one or more estimated glucose levels. In some aspects, the updated conversion function may have one or more updated lag parameters. In some aspects, one or more updated lag parameters may include one or more of an updated analyte diffusion rate ($p_2$) and an updated analyte consumption rate ($p_3$). In some aspects, the calibration process 600 may proceed from step 612 to step 602 (or to step 614 as shown in FIG. 6), and the transceiver 101 may use the updated conversion function to calculate glucose levels from subsequent sensor data.

In some aspects, the calibration process 600 may include the step 614 in which the transceiver 101 determines whether the transceiver 101 has received an updated error model. In some aspects, the transceiver 101 may receive an updated error model from the display device 105 (e.g., via the display interface of the transceiver 101). In some aspects, the error model may have been updated based on one or more new experimental datasets. In some aspects, the updated error model may model differences between capillary blood glucose level measurements and venous blood glucose level measurements. In some aspects, if the transceiver 101 has not received an updated error model, the calibration process 600 may proceed to step 602. In some aspects, if the transceiver 101 has received a reference measurement, the calibration process 600 may proceed to a step 616.

In some aspects, the calibration process 600 may include the step 616 in which the transceiver 101 replaces the error model used to convert the received reference measurements (capillary blood glucose measurements) into estimated glucose levels (estimated venous blood glucose levels) with the received updated error model. In some aspects, the calibration process 600 may proceed from step 616 to step 602, and the transceiver 101 may use the updated error model to convert subsequent reference measurements into estimated glucose levels.

In some aspects, as described above, the transceiver 101 may convert the received reference glucose measurement (i.e., a reference capillary blood glucose measurement such as an SMBG or finger-stick measurement) into an estimated glucose level (i.e., an estimated venous blood glucose level). In some aspects, the conversion may occur, for example, in step 610 of the calibration process 600. The conversion of the reference glucose measurement into the estimated glucose level is based on a model (e.g., a linear regression model) of differences between capillary blood glucose level measurements and venous blood glucose level measurements.

According to the disclosure instead of using a single model over the entire range, different models are used to convert reference glucose measurements over different ranges of reference glucose measurements into estimated glucose levels. Different first, second, and third models are used to convert reference glucose measurements over first, second, and third ranges of reference glucose measurements (i.e., 40-69 mg/dL, 70-179 mg/dL, and 180-400 mg/dL for capillary blood glucose level measurements), respectively, into estimated glucose levels. That is, one of multiple models is selected based on an glucose level range into which the reference glucose measurement falls, and the selected model is used to convert the reference glucose measurement into an estimated glucose level. Use of different models over different glucose level ranges may improve the accuracy of the estimation. Each of the multiple models that is used for conversion of a sub-range of reference glucose measurements may provide more accurate estimated glucose levels for the sub-range of reference glucose measurements than the single model that is used for the entire range reference glucose measurements.

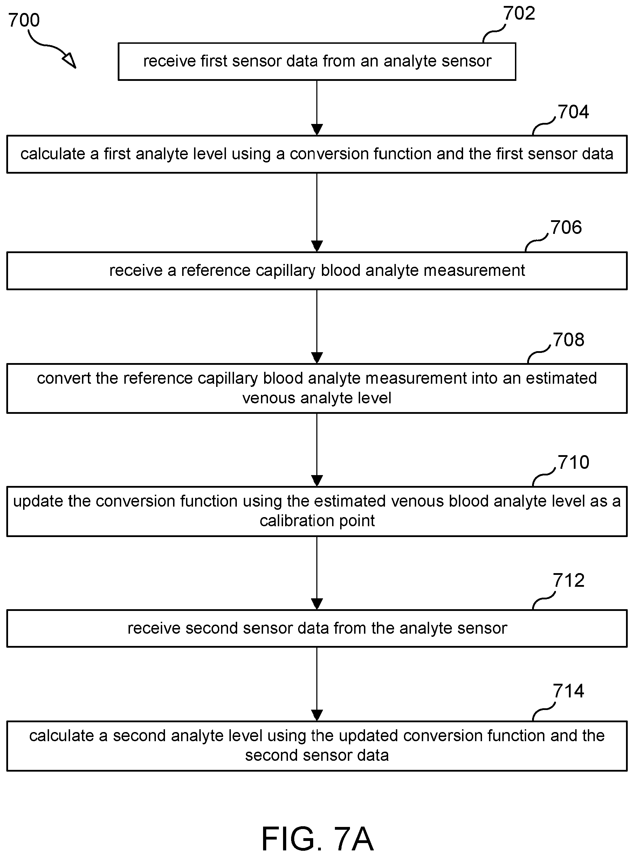

FIG. 7A illustrates a calibration process 700 according to some different models for different ranges aspects. In some aspects, one or more of the steps of the process 700 may be performed by the transceiver 101 (e.g., the computer 920 of the transceiver 101).

As shown in FIG. 7A, the process 700 includes a step 702 of using the transceiver 101 to receive first sensor data from an analyte sensor 100. In some aspects, the first sensor data may include light and temperature measurements.

As shown in FIG. 7A, the process 700 includes a step 704 of using the transceiver 101 to calculate a first glucose level using a conversion function and the first sensor data. In some aspects, the first glucose level may be a second medium glucose level. In some aspects, calculating the first glucose level using the conversion function and the first sensor data may include: (i) calculating a first medium glucose level using at least the first sensor data; (ii) calculating a first medium glucose level rate of change using at least the first medium glucose level; and (iii) calculating the second medium glucose level using at least the first medium glucose level and first medium glucose level rate of change. In some aspects, the first medium may be interstitial fluid, and the second medium may be blood.

As shown in FIG. 7A, the process 700 includes a step 706 of using the transceiver 101 to receive a reference capillary blood glucose measurement. In some aspects, the reference capillary blood glucose measurement may be a self-monitoring blood glucose (SMBG) measurement obtained from a finger-stick blood sample.

As shown in FIG. 7A, the process 700 includes a step 708 of using the transceiver 101 to convert the reference capillary blood glucose measurement into an estimated venous glucose level.

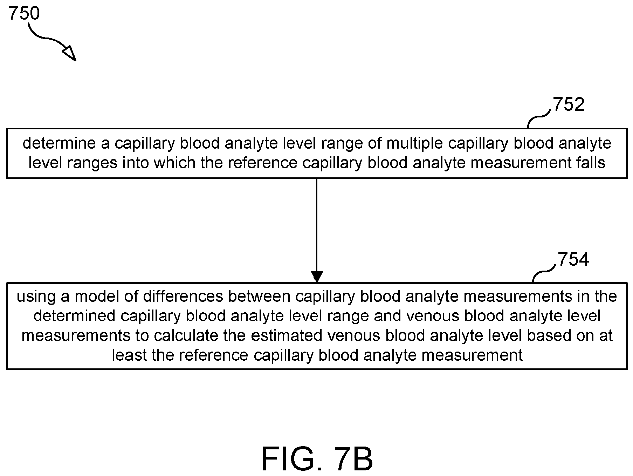

FIG. 7B illustrates a conversion process 750 that is performed during the step 708 of converting the reference capillary blood glucose measurement into the estimated venous blood glucose level. In some aspects, as shown in FIG. 7B, the process 750 includes a step 752 in which the transceiver 101 determines a capillary blood glucose level range of multiple capillary blood glucose level ranges into which the reference capillary blood glucose measurement falls. As shown in FIG. 7B, the process 750 includes a step 754 in which the transceiver 101 uses a model of differences between capillary blood glucose measurements in the determined capillary blood glucose level range and venous blood glucose level measurements to calculate the estimated venous blood glucose level based on at least the reference capillary blood glucose measurement. The multiple capillary blood glucose level ranges do not overlap with one another. The multiple capillary blood glucose level ranges include three non-overlapping capillary blood glucose level ranges (i.e., 40-69 mg/dL, 70-179 mg/dL, and 180-400 mg/dL for capillary blood glucose level measurements).

In some aspects, the conversion of the reference capillary blood glucose measurement into the estimated venous blood glucose level may be based on a cost function that maximizes the likelihood of a fit of an error between calculated glucose levels and capillary blood glucose level measurements to the model of the differences between the capillary blood glucose level measurements in the determined capillary blood glucose level range and the venous blood glucose level measurements. In some aspects, the conversion of the reference glucose measurement into the estimated glucose level may be based on a cost function that minimizes the negative likelihood of a fit of an error between calculated glucose levels and capillary blood glucose level measurements to the model of the differences between the capillary blood glucose level measurements in the determined capillary blood glucose level range and the venous blood glucose level measurements.

As shown in FIG. 7A, the process 700 includes a step 710 of using the transceiver to update the conversion function using the estimated venous blood glucose level as a calibration point. In some aspects, the updated conversion function may minimize an error between glucose levels calculated using the updated conversion function and estimated venous blood glucose levels.

As shown in FIG. 7A, the process 700 includes a step 712 of using the transceiver 101 to receive second sensor data from the analyte sensor.

As shown in FIG. 7A, the process 700 includes a step 714 of using the transceiver 101 to calculate a second glucose level using the updated conversion function and the second sensor data.

In some aspects, the process 700 may further include steps of: (i) using the transceiver 101 to receive an updated model of differences between capillary blood glucose measurements in the determined capillary blood glucose level range and venous blood glucose level measurements; (ii) using the transceiver 101 to receive a second capillary blood reference glucose measurement in the determined capillary blood glucose level range; (iii) using the transceiver 101 to convert the second reference capillary blood glucose measurement into a second estimated venous blood glucose level using the updated model; (iv) using the transceiver 101 to update the conversion function using the second estimated venous blood glucose level as a calibration point; (v) using the transceiver 101 to receive third sensor data from the analyte sensor 100;

and (vi) using the transceiver 101 to use the twice updated conversion function and the third sensor data to calculate a third glucose level.

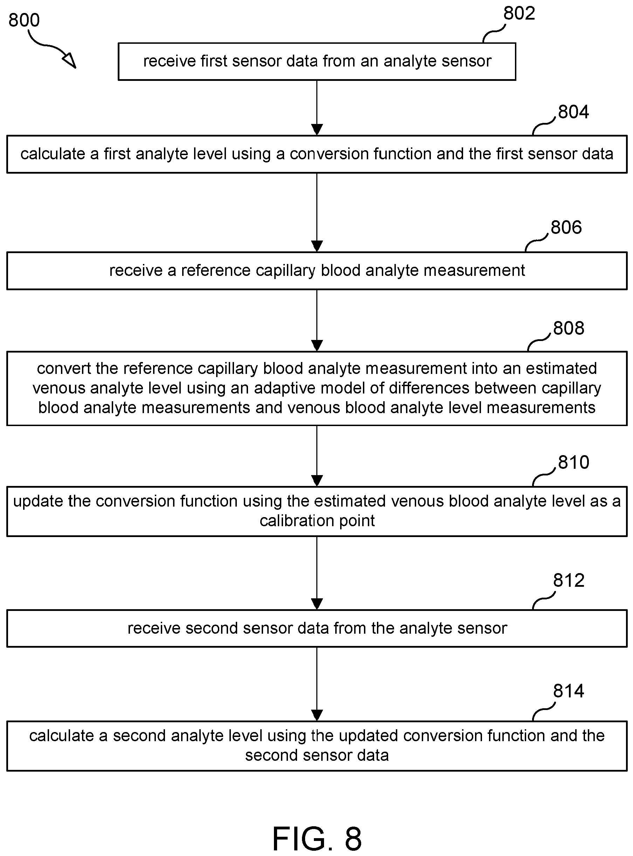

FIG. 8 which is not part of the invention, illustrates a calibration process 800 according to some adaptive model aspects. In some aspects, one or more of the steps of the process 800 may be performed by the transceiver 101 (e.g., the computer 920 of the transceiver 101).

In some aspects, as shown in FIG. 8, the process 800 may include step 802 of using a transceiver 101 to receive first sensor data from an analyte sensor 100, a step 804 of using the transceiver 101 to calculate a first glucose level using a conversion function and the first sensor data, and a step 806 of using the transceiver 101 to receive a reference capillary blood glucose measurement. **In** some aspects, the steps 802, 806, and 808 may correspond to the steps 702, 704, and 706, respectively, of the calibration process 700, which is described above.

**In** some aspects, as shown in FIG. 8, the process 800 may include a step 808 using the transceiver 101 to convert the reference capillary blood glucose measurement into an estimated venous glucose level using an adaptive model of differences between capillary blood glucose measurements and venous blood glucose level measurements. **In** some aspects, one or more parameters of the adaptive model may change depending on the reference capillary blood glucose measurement. **In** some aspects, a structure of the adaptive model may additionally or alternatively change depending on the reference capillary blood glucose measurement (e.g., as in the case of a decision tree).

In some aspects, the conversion of the reference capillary blood glucose measurement into the estimated venous blood glucose level in step 808 may be based on a cost function that maximizes the likelihood of a fit of an error between calculated glucose levels and capillary blood glucose level measurements to the adaptive model of the differences between the capillary blood glucose level measurements and the venous blood glucose level measurements. In some aspects, the conversion of the reference capillary blood glucose measurement into the estimated venous blood glucose level in step 808 may be based on a cost function that minimizes the negative likelihood of a fit of an error between calculated glucose levels and capillary blood glucose level measurements to the model of the differences between the capillary blood glucose level measurements in the determined capillary blood glucose level range and the venous blood glucose level measurements.

In some aspects, as shown in FIG. 8, the process 800 may include a step 810 of using the transceiver 101 to update the conversion function using the estimated venous blood glucose level as a calibration point, a step 812 of using the transceiver 812 to receive second sensor data from the analyte sensor 100, and a step 814 of using the transceiver 101 to calculate a second glucose level using the updated conversion function and the second sensor data. In some aspects, the steps 810, 812, and 814 may correspond to the steps 710, 712, and 714, respectively, of the calibration process 700, which is described above.

In some aspects, the process 800 may further include: (i) using the transceiver 101 to receive an updated adaptive model of differences between capillary blood glucose measurements and venous blood glucose level measurements; (ii) using the transceiver 101 to receive a second capillary blood reference glucose measurement in the determined capillary blood glucose level range; (iii) using the transceiver 101 to convert the second reference capillary blood glucose measurement into a second estimated venous blood glucose level using the updated model; (iv) using the transceiver 101 to update the conversion function using the second estimated venous blood glucose level as a calibration point; (v) using the transceiver 101 to receive third sensor data from the analyte sensor; and (vi) using the transceiver 101 to use the twice updated conversion function and the third sensor data to calculate a third glucose level.

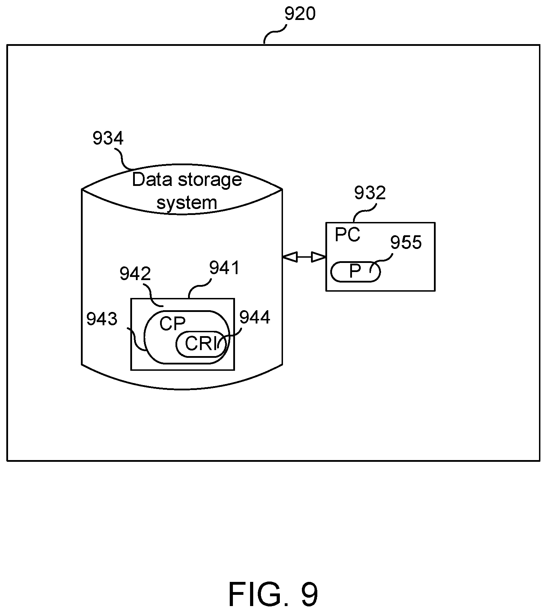

FIG. 9 is a block diagram of the computer 920 of the transceiver 101 according to some aspects. As shown in FIG. 9, the computer 920 may include: processing circuitry (PC) 932, which may include one or more processors (P) 955 (e.g., one or more general purpose microprocessors and/or one or more other processors, such as an application specific integrated circuit (ASIC), field-programmable gate arrays (FPGAs), and the like); and a local storage unit (a.k.a., "data storage system") 934, which may include one or more non-volatile storage devices and/or one or more volatile storage devices. In aspects in which the PC 932 includes a programmable processor, the local storage unit 934 may include a computer program product (CPP) 941. In some aspects, the CPP 941 may include a computer readable medium (CRM) 942 storing a computer program (CP) 943 including computer readable instructions (CRI) 944. In some aspects, the CRM 942 may be a non-transitory computer readable medium, such as, magnetic media (e.g., a hard disk), optical media, memory devices (e.g., random access memory, flash memory), and the like. In some aspects, the CRI 944 of computer program 943 may be configured such that when executed by PC 902, the CRI causes the computer 920 to perform steps described herein (e.g., one or more steps described herein with reference to the flowcharts herein). In some other aspects, the computer 920 may be configured to perform steps described herein without the need for code. That is, for example, the PC 932 may consist merely of one or more ASICs. Hence, the features of the aspects described herein may be implemented in hardware and/or software.

Aspects of the present disclosure have been fully described above with reference to the drawing figures. For example, although the disclosure is described above in the context of an analyte monitoring system that calculates blood glucose levels indirectly using measurements of glucose levels in interstitial fluid, the disclosure is applicable to any monitoring system that calculates levels in a first medium using measurements of levels in a second medium. Systems,

**EP 4 335 368 B1**

methods, and apparatuses for reducing error between calculated glucose levels and impractical glucose measurements using practical glucose measurements as reference measurements for calibration. Reducing the error may include converting a practical reference glucose measurement into an estimated impractical glucose level, updating a conversion function using the estimated impractical glucose level, and using the updated conversion function to calculate an glucose level. The practical reference glucose measurement is a capillary blood glucose measurement, and the estimated impractical glucose level is an estimated venous blood glucose level. In some aspects, the updated conversion function may minimize the error between glucose levels calculated using the updated conversion function and estimated venous blood glucose levels.

**Claims**

1. A system (50) comprising:

   an analyte sensor (100); and
   a transceiver (101) configured to:

      receive first sensor data from the analyte sensor;
      calculate a first glucose level using a conversion function and the first sensor data;
      receive a reference capillary blood glucose measurement;
      convert the reference capillary blood glucose measurement into an estimated venous blood glucose level;
      update the conversion function using the estimated venous blood glucose level as a calibration point;
      receive second sensor data from the analyte sensor; and
      use the updated conversion function and the second sensor data to calculate a second glucose level;
      **characterized in that**, in converting the reference capillary blood glucose measurement into the estimated venous blood glucose level, the transceiver is configured to:

         determine a capillary blood glucose level range of multiple capillary blood glucose level ranges into which the reference capillary blood glucose measurement falls, wherein the multiple capillary blood glucose level ranges do not overlap with one another, and the multiple capillary blood glucose level ranges include a first range from 40-69 mg/dL, a second range from 70-179 mg/dL, and a third range from 180-400 mg/dL;
         select one of first, second, and third models based on the determined capillary blood glucose level range, wherein the first model is a model of differences between capillary blood glucose measurements in the first range and venous blood glucose measurements, the first model is selected if the reference capillary blood glucose measurement is determined to fall into the first range, the second model is a model of differences between capillary blood glucose measurements in the second range and venous blood glucose measurements, the second model is selected if the reference capillary blood glucose measurement is determined to fall into the second range, a third model is a model of differences between capillary blood glucose measurements in the third range and venous blood glucose measurements, and the third model is selected if the reference capillary blood glucose measurement is determined to fall into the third range; and
         use the selected one of the first, second, and third models to calculate the estimated venous blood glucose level based on at least the reference capillary blood glucose measurement.

2. The system of claim 1, wherein the transceiver is further configured to:

   receive an updated model of differences between capillary blood glucose measurements in the determined capillary blood glucose level range and venous blood glucose level measurements;
   receive a second reference capillary blood glucose measurement in the determined capillary blood glucose level range;
   convert the second reference capillary blood glucose measurement into a second estimated venous blood glucose level using the updated model;
   update the conversion function using the second estimated venous blood glucose level as a calibration point;
   receive third sensor data from the analyte sensor; and
   use the twice updated conversion function and the third sensor data to calculate a third glucose level.

3. The system of claim 1 or 2, wherein the updated conversion function minimizes an error between glucose levels

13

calculated using the updated conversion function and estimated venous blood glucose levels.

4. The system of any one of claims 1-3,
wherein the reference capillary blood glucose measurement is a self-monitoring blood glucose (SMBG) measurement obtained from a finger-stick blood sample.

5. The system of any one of claims 1-4, wherein the first sensor data includes light and temperature measurements.

6. The system of any one of claims 1-5, wherein the conversion of the reference capillary blood glucose measurement into the estimated venous blood glucose level is based on a cost function that maximizes the likelihood or minimizes the negative likelihood of a fit of an error between calculated glucose levels and capillary blood glucose level measurements to the selected one of the first, second, and third models.

7. The system of any one of claims 1-6, wherein the first glucose level is a second medium glucose level, and calculating the first glucose level using the conversion function and the first sensor data comprises:

calculating a first medium glucose level using at least the first sensor data;
calculating a first medium glucose level rate of change using at least the first medium glucose level; and
calculating the second medium glucose level using at least the first medium glucose level and first medium glucose level rate of change.

8. The system of claim 7, wherein the first medium is interstitial fluid, and the second medium is blood.

**Patentansprüche**

1. System (50), umfassend:

einen Analyt-Sensor (100); und
einen Transceiver (101), der so konfiguriert ist, dass er:

erste Sensordaten von dem Analyt-Sensor empfängt;
einen ersten Glukosespiegel unter Verwendung einer Umwandlungsfunktion und der ersten Sensordaten berechnet;
eine Referenz-Kapillarblutglukosemessung empfängt;
die Referenz-Kapillarblutglukosemessung in einen geschätzten venösen Blutglukosespiegel umwandelt;
die Umwandlungsfunktion unter Verwendung des geschätzten venösen Blutglukosespiegels als Kalibrierungspunkt aktualisiert;
zweite Sensordaten von dem Analyt-Sensor empfängt; und
die aktualisierte Umwandlungsfunktion und die zweiten Sensordaten verwendet, um einen zweiten Glukosespiegel zu berechnen;
**dadurch gekennzeichnet, dass** der Transceiver bei der Umwandlung der Referenz-Kapillarblutglukosemessung in den geschätzten venösen Blutglukosespiegel so konfiguriert ist, dass er:

einen Kapillarblutglukosespiegelbereich aus mehreren Kapillarblutglukosespiegelbereichen ermittelt, in den die Referenz-Kapillarblutglukosemessung fällt, wobei die mehreren Kapillarblutglukosespiegelbereiche nicht miteinander überlappen, und die mehreren Kapillarblutglukosespiegelbereiche einen ersten Bereich von 40-69 mg/dl, einen zweiten Bereich von 70-179 mg/dl und einen dritten Bereich von 180-400 mg/dl umfassen;
ein erstes, zweites oder drittes Modell auf der Grundlage des ermittelten Kapillarblutglukosespiegelbereichs auswählt, wobei das erste Modell ein Modell von Unterschieden zwischen Kapillarblutglukosemessungen im ersten Bereich und venösen Blutglukosemessungen ist, das erste Modell ausgewählt wird, wenn festgestellt wird, dass die Referenz-Kapillarblutglukosemessung in den ersten Bereich fällt, das zweite Modell ein Modell von Unterschieden zwischen Kapillarblutglukosemessungen im zweiten Bereich und venösen Blutglukosemessungen ist, das zweite Modell ausgewählt wird, wenn festgestellt wird, dass die Referenz-Kapillarblutglukosemessung in den zweiten Bereich fällt, ein drittes Modell ein Modell von Unterschieden zwischen Kapillarblutglukosemessungen im dritten Bereich und venösen Blutglukosemessungen ist, und das dritte Modell ausgewählt wird, wenn festgestellt wird, dass die Referenz-Kapillarblutglukosemessung in den dritten Bereich fällt; und

das ausgewählte erste, zweite oder dritte Modell verwendet, um den geschätzten venösen Blutglukosespiegel auf der Grundlage mindestens der Referenz-Kapillarblutglukosemessung zu berechnen.

2. System nach Anspruch 1, wobei der Transceiver ferner so konfiguriert ist, dass er:

ein aktualisiertes Modell von Unterschieden zwischen Kapillarblutglukosemessungen im ermittelten Kapillarblutglukosespiegelbereich und venösen Blutglukosespiegelmessungen empfängt;

eine zweite Referenz-Kapillarblutglukosemessung im ermittelten Kapillarblutglukosespiegelbereich empfängt;

die zweite Referenz-Kapillarblutglukosemessung unter Verwendung des aktualisierten Modells in einen zweiten geschätzten venösen Blutglukosespiegel umwandelt;

die Umwandlungsfunktion unter Verwendung des zweiten geschätzten venösen Blutglukosespiegels als Kalibrierungspunkt aktualisiert;

dritte Sensordaten von dem Analyt-Sensor empfängt; und

die zweimal aktualisierte Umwandlungsfunktion und die dritten Sensordaten verwendet, um einen dritten Glukosespiegel zu berechnen.

3. System nach Anspruch 1 oder 2, wobei die aktualisierte Umwandlungsfunktion einen Fehler zwischen Glukosespiegeln, die unter Verwendung der aktualisierten Umwandlungsfunktion berechnet werden und geschätzten venösen Blutglukosespiegeln minimiert.

4. System nach einem der Ansprüche 1-3,
wobei die Referenz-Kapillarblutglukosemessung eine Selbstkontroll-Blutglukosemessung (SMBG) ist, die aus einer Fingerstich-Blutprobe gewonnen wird.

5. System nach einem der Ansprüche 1-4, wobei die ersten Sensordaten Licht- und Temperaturmessungen beinhalten.

6. System nach einem der Ansprüche 1-5, wobei die Umwandlung der Referenz-Kapillarblutglukosemessung in den geschätzten venösen Blutglukosespiegel auf einer Kostenfunktion basiert, die die Wahrscheinlichkeit einer Anpassung eines Fehlers zwischen berechneten Glukosespiegeln und Kapillarblutglukosespiegelmessungen an das ausgewählte erste, zweite oder dritte Modell maximiert oder die negative Wahrscheinlichkeit minimiert.

7. System nach einem der Ansprüche 1-6, wobei der erste Glukosespiegel ein Glukosespiegel in einem zweiten Medium ist und das Berechnen des ersten Glukosespiegels unter Verwendung der Umwandlungsfunktion und der ersten Sensordaten umfasst:

Berechnen eines Glukosespiegels in einem ersten Medium unter Verwendung mindestens der ersten Sensordaten;

Berechnen einer Glukosespiegel-Änderungsrate im ersten Medium unter Verwendung mindestens des Glukosespiegel im ersten Medium; und

Berechnen des Glukosespiegels im zweiten Medium unter Verwendung mindestens des Glukosespiegels im ersten Medium und der Glukosespiegel-Änderungsrate im ersten Medium.

8. System nach Anspruch 7, wobei das erste Medium Interstitialfluid ist und das zweite Medium Blut ist.

**Revendications**

1. Système (50) comprenant :

un capteur d'analyte (100) ; et
un émetteur-récepteur (101) configuré pour :

recevoir des premières données de capteur du capteur d'analyte ;
calculer un premier taux de glucose à l'aide d'une fonction de conversion et des premières données de capteur ;
recevoir une mesure de glycémie capillaire de référence ;
convertir la mesure de glycémie capillaire de référence en un taux de glycémie veineuse estimé ;

mettre à jour la fonction de conversion à l'aide du taux de glycémie veineuse estimé comme point d'étalonnage ;

recevoir des deuxièmes données de capteur du capteur d'analyte ; et

utiliser la fonction de conversion mise à jour et les deuxièmes données de capteur pour calculer un deuxième taux de glucose ;

**caractérisé en ce que**, en convertissant la mesure de glycémie capillaire de référence en taux de glycémie veineuse estimé, l'émetteur-récepteur est configuré pour :

déterminer une plage de taux de glycémie capillaire parmi de multiples plages de taux de glycémie capillaire dans laquelle s'inscrit la mesure de glycémie capillaire de référence, dans lequel les multiples plages de taux de glycémie capillaire ne se chevauchent pas, et les multiples plages de taux de glycémie capillaire incluent une première plage de 40-69 mg/dL, une deuxième plage de 70-179 mg/dL et une troisième plage de 180-400 mg/dL ;

sélectionner l'un parmi des premier, deuxième et troisième modèles sur la base de la plage de taux de glycémie capillaire déterminée, dans lequel le premier modèle est un modèle de différences entre les mesures de glycémie capillaire dans la première plage et les mesures de glycémie veineuse, le premier modèle est sélectionné si la mesure de glycémie capillaire de référence est déterminée pour s'inscrire dans la première plage, le deuxième modèle est un modèle de différences entre des mesures de glycémie capillaire dans la deuxième plage et des mesures de glycémie veineuse, le deuxième modèle est sélectionné si la mesure de glycémie capillaire de référence est déterminée pour s'inscrire dans la deuxième plage, un troisième modèle est un modèle de différences entre des mesures de glycémie capillaire dans la troisième plage et des mesures de glycémie veineuse, et le troisième modèle est sélectionné si la mesure de glycémie capillaire de référence est déterminée pour s'inscrire dans la troisième plage ; et

utiliser le modèle sélectionné parmi les premier, deuxième et troisième modèles pour calculer le taux de glycémie veineuse estimé sur la base d'au moins la mesure de glycémie capillaire de référence.

2. Système selon la revendication 1, dans lequel l'émetteur-récepteur est en outre configuré pour :

recevoir un modèle mis à jour de différences entre les mesures de glycémie capillaire dans la plage de taux de glycémie capillaire déterminée et les mesures de taux de glycémie veineuse ;

recevoir une seconde mesure de glycémie capillaire de référence dans la plage de taux de glycémie capillaire déterminée ;

convertir la seconde mesure de glycémie capillaire de référence en un second taux de glycémie veineuse estimé à l'aide du modèle mis à jour ;

mettre à jour la fonction de conversion à l'aide du second taux de glycémie veineuse estimé comme point d'étalonnage ;

recevoir des troisièmes données de capteur du capteur d'analyte ; et

utiliser la fonction de conversion mise à jour deux fois et les troisièmes données de capteur pour calculer un troisième taux de glucose.

3. Système selon la revendication 1 ou 2, dans lequel la fonction de conversion mise à jour minimise une erreur entre les taux de glucose calculés à l'aide de la fonction de conversion mise à jour et les taux de glycémie veineuse estimés.

4. Système selon l'une quelconque des revendications 1-3,
dans lequel la mesure de glycémie capillaire de référence est une mesure de glycémie autosurveillée (SMBG) obtenue à partir d'un échantillon de sang prélevé au doigt.

5. Système selon l'une quelconque des revendications 1-4, dans lequel les premières données de capteur incluent des mesures de lumière et de température.

6. Système selon l'une quelconque des revendications 1-5, dans lequel la conversion de la mesure de glycémie capillaire de référence en taux de glycémie veineuse estimé est basée sur une fonction de coût qui maximise la probabilité ou minimise la probabilité négative d'un ajustement d'une erreur entre les taux de glucose calculés et les mesures de taux de glycémie capillaire au modèle sélectionné parmi les premier, deuxième et troisième modèles.

7. Système selon l'une quelconque des revendications 1-6, dans lequel le premier taux de glucose est un taux de glucose de second milieu, et le calcul du premier taux de glucose à l'aide de la fonction de conversion et des premières données de capteur comprend :

le calcul d'un taux de glucose de premier milieu à l'aide d'au moins les premières données de capteur ;
le calcul d'une vitesse de changement de taux de glucose de premier milieu à l'aide d'au moins le taux de glucose de premier milieu ; et
le calcul du taux de glucose de second milieu à l'aide d'au moins le taux de glucose de premier milieu et de la vitesse de changement de taux de glucose de premier milieu.

8. Système selon la revendication 7, dans lequel le premier milieu est un fluide interstitiel, et le second milieu est du sang.

Sensor
(subcutaneous)
100

13.56 MHz

Power &
Data

Body-Worn
Transmitter
101

Bluetooth Low
Energy

8:54 AM

Current Glucose

124 mg/dL

TODAY
10AM  2PM  8PM  12AM  6AM

180

60

Log Event        Menu

Smartphone
with Mobile
Medical App
105

FIG. 1

EP 4 335 368 B1

FIG. 2

FIG. 3

FIG. 4

FIG. 5

EP 4 335 368 B1

600

start

602 receive sensor data?

602

Y → 604 calculate analyte level

N

606 receive RM?

Y → store RM 608

N

610 convert RM

612 calibrate using converted RM

614 receive updated error model?

Y → 616 update error model

N

FIG. 6

700

702

receive first sensor data from an analyte sensor

704

calculate a first analyte level using a conversion function and the first sensor data

706

receive a reference capillary blood analyte measurement

708

convert the reference capillary blood analyte measurement into an estimated venous analyte level

710

update the conversion function using the estimated venous blood analyte level as a calibration point

712

receive second sensor data from the analyte sensor

714

calculate a second analyte level using the updated conversion function and the second sensor data

FIG. 7A

750

752

determine a capillary blood analyte level range of multiple capillary blood analyte
level ranges into which the reference capillary blood analyte measurement falls

754

using a model of differences between capillary blood analyte measurements in the
determined capillary blood analyte level range and venous blood analyte level
measurements to calculate the estimated venous blood analyte level based on at
least the reference capillary blood analyte measurement

# FIG. 7B

800

802

receive first sensor data from an analyte sensor

804

calculate a first analyte level using a conversion function and the first sensor data

806

receive a reference capillary blood analyte measurement

808

convert the reference capillary blood analyte measurement into an estimated venous analyte level using an adaptive model of differences between capillary blood analyte measurements and venous blood analyte level measurements

810

update the conversion function using the estimated venous blood analyte level as a calibration point

812

receive second sensor data from the analyte sensor

814

calculate a second analyte level using the updated conversion function and the second sensor data

# FIG. 8

920

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021108610 A1
- EP 2710951 A1, UNIV VALENCIA POLITECNICA [ES]
- US 2005187720 A1, GOODE PAUL V JR [US]
- US 76183913
- US 93787113
- US 65001612
- US 761839
- US 13937871
- US 13650016

### Non-patent literature cited in the description

- **PAOLO ROSSETTI et al.** Estimating Plasma Glucose from Interstitial Glucose: The Issue of Calibration Algorithms in Commercial Continuous Glucose Monitoring Devices. *SENSORS*, 03 December 2010, vol. 10 (12), 10936-10952